# EUROPÄISCHE PATENTANMELDUNG

(11) **EP 4 843 595 A1**
(43) Veröffentlichungstag der Anmeldung: **25.11.2026**
(21) Anmeldenummer: 25177362.8
(22) Anmeldetag: 19.05.2025
(51) Int. Cl.: A61M 5/36

(54) **ULTRASCHALLSENSOR ZUR LUFTBLASENERKENNUNG IN INFUSIONSFLÜSSIGKEITEN**

(71) Anmelder: B. Braun Melsungen AG, 34212 Melsungen (DE)
(72) Erfinder: BRESSAU, Ernst, 34323 Malsfeld (DE); HOLLSTEIN, Rolf, 36211 Alheim-Heinebach (DE)
(74) Vertreter: Winter, Brandl - Partnerschaft mbB

(57) **Zusammenfassung**

Offenbart sind ein Luftblasensensor (102; 202) und eine damit ausgerüstete Infusionspumpe (101; 201). Der Luftblasensensor (102; 202) hat einen Ultraschallsender (7) und einen Ultraschallempfänger (9) und eine Schlauchaufnahme, die sich entlang einer etwa waagerechten und/oder horizontalen Förderrichtung (11) erstreckt. Der Ultraschallsender (7) ist an einer ersten Seite der Schlauchaufnahme angeordnet oder er bildet eine erste Seite der Schlauchaufnahme. Der Ultraschallempfänger (9) an einer der ersten Seite gegenüberliegenden zweiten Seite angeordnet ist oder er bildet eine zweite Seite der Schlauchaufnahme. Eine vom Ultraschallsender (7) zum Ultraschallempfänger (9) gerichtete Messtrecke (112) ist nicht vertikal bzw. nicht senkrecht, bevorzugt maximal 45°, vorzugsweise maximal 25° zur Horizontalen und/oder Waagerechten angestellt.

## Beschreibung

### Technisches Gebiet

Die vorliegende Offenbarung betrifft die Luftblasenerkennung und vorzugsweise eine zusätzliche Partikelerkennung einer medizinischen Infusionsflüssigkeit in einer Infusionsleitung, vorzugsweise in einem Infusionsschlauch einer Infusionspumpe mittels einem Ultraschallsensor. Genauer gesagt betrifft die vorliegende Offenbarung einen Ultraschallsensor und eine Infusionspumpe mit einem Ultraschallsensor.

### Hintergrund der Offenbarung

In der Medizin werden flüssige Medikamente mittels Infusionspumpen einem Patienten zugeführt und in dessen Blutkreislauf konstant oder schubweise injiziert. Dabei ist es wichtig, dass keine Luftblasen (oder andere Gasblasen) zusammen mit dem flüssigen Medikament eingebracht werden. Das flüssige Medikament wird im Folgenden als Infusionsflüssigkeit bezeichnet.

### Stand der Technik

In der EP 3 296 717 A1 der Anmelderin ist ein optischer bildgebender Sensor am Außenumfang eines Schlauches einer Dialysemaschine offenbart, wobei der Sensor Fremdstoffe wie Verunreinigungen und Luftblasen im Schlauch erkennen soll.

Aus dem Stand der Technik sind peristaltische Infusionspumpen bekannt, in deren Schlauchaufnahme(n) ein Schlauchabschnitt eines Infusionsschlauches geradlinig eingelegt wird. Die so genannte Peristaltik hat mehrere äußere radial bewegliche Verdrängungskörper, die aufeinander folgend derart in den elastischen Schlauch (oder ein extra gebildetes elastisches Pumpensegment) des Infusionsschlauches eingedrückt werden, dass die Infusionsflüssigkeit weitergefördert wird. Benachbart zur Peristaltik ist ein Ultraschallsensor zur Luftblasenerkennung vorgesehen.

Gemäß dem Stand der Technik haben derartige Infusionspumpen an einer vorderen Einlegeseite eine Frontklappe, die geöffnet wird, bevor der betroffene Schlauchabschnitt des Infusionsschlauches in waagerechter bzw. horizontaler Richtung in mehrere nach vorne geöffnete Schlauchaufnahmen (Schlauchbefestigungen) eingelegt wird. Danach wird die vordere Einlegeseite der Infusionspumpe mit der Frontklappe geschlossen, wobei die Fronklappe dazu beiträgt, den betroffenen Schlauchabschnitt des Infusionsschlauches gehäusefest zu halten und zu sichern.

Der direkt hinter der Frontklappe angeordnete Luftblasensensor hat einen Ultraschallsender und einen Ultraschallempfänger am Außenumfang des Schlauches. Passend zu der oben beschriebenen horizontalen Einlegerichtung des Infusionsschlauches sind der Sender und der Empfänger vertikal, also entlang der Schwerkraftrichtung übereinander angeordnet. Damit ergibt sich gemäß dem Stand der Technik eine senkrechte Ultraschall-Messstrecke mittig durch den Infusionsschlauch.

Insbesondere in dem waagerecht bzw. horizontal verlaufenden Schlauchabschnitt des Infusionsschlauches können sich so genannte stehende Luftblasen an der Schlauchinnenwand festsetzen, die von der strömenden Infusionsflüssigkeit für eine signifikante Zeit nicht mitgenommen werden. Dieses Festsetzen kann z.B. so lange dauern, bis eine weitere Luftblase (Putzerblase), die sich zusammen mit der strömenden Infusionsflüssigkeit bewegt, die betroffene stehende Luftblase mitreißt.

Da die Luft oder das andere Gas, welche(s) die Blase bildet, eine geringere Dichte als die Infusionsflüssigkeit hat, entstehen die stehenden Luftblasen in der Regel an der Oberseite des Infusionsschlauches, also oben an der Schlauchinnenwand.

Wenn sich nun eine stehende Luftblase (zufälligerweise) zwischen dem Ultraschallsender und dem Ultraschallempfänger festsetzt, leitet eine Elektronik bzw. eine Steuereinheit der Infusionspumpe aus der daraus folgenden andauernden Dämpfung des Ultraschallsignals fälschlicherweise ab, dass eine große Menge Luft in der zum Patienten strömenden Infusionsflüssigkeit vorhanden ist und löst einen Fehlalarm aus.

### Kurzbeschreibung der Offenbarung

Aufgabe der vorliegenden Offenbarung ist es, einen auf Ultraschall basierenden Luftblasensensor und eine Infusionspumpe mit einem derartigen Luftblasensensor zu schaffen, bei denen die Luftblasenerkennung verbessert ist. Insbesondere soll der Fehlalarm bei einer unkritischen stehenden Luftblase im Messkanal zwischen dem Ultraschallsender und dem Ultraschallempfänger vermieden werden.

Diese Aufgabe wird gelöst durch einen Luftblasensensor mit den Merkmalen des Anspruchs 1 und durch eine Infusionspumpe mit den Merkmalen des Anspruchs 9.

Der Luftblasensensor gemäß der Offenbarung hat einen Ultraschallsender und einen Ultraschallempfänger und eine Schlauchaufnahme, die zur Aufnahme eines Infusionsschlauches vorbereitet und eingerichtet ist. Die Schlauchaufnahme erstreckt sich insbesondere entlang einer etwa waagerechten und/oder horizontalen Förderrichtung, die Schlauchaufnahme ist also zum Beispiel maximal 5° zur Waagerechten oder Horizontalen angestellt. In anderen Worten ist die Schlauchaufnahme derart ausgebildet, dass ein in ihr aufgenommener Schlauch horizontal bzw. waagerecht ausgerichtet ist. Die Adjektive "horizontal" und "waagerecht" beziehen sich auf das Schwerefeld der Erde. Mit dieser Anordnung der Schlauchaufnahme und des darin aufnehmbaren Infusionsschlauches ergibt sich das Problem der möglicherweise im Infusionsschlauch stehenden Luftblasen. Der Ultraschallsender ist an einer ersten Seite der Schlauchaufnahme angeordnet und/oder bildet eine erste Seite der Schlauchaufnahme. Der Ultraschallempfänger ist an einer der ersten Seite gegenüberliegenden zweiten Seite der Schlauchaufnahme angeordnet und/oder bildet diese zweite Seite der Schlauchaufnahme. Dabei sind Ultraschallsender und Ultraschallempfänger seitlich am Außenumfang des aufnehmbaren Infusionsschlauches angeordnet. Eine vom Ultraschallsender zum Ultraschallempfänger gerichtete Messtrecke ist nicht vertikal bzw. nicht senkrecht (zur Erdoberfläche), bevorzugt maximal 45°, besonders bevorzugt maximal 25° zur Horizontalen und/oder Waagerechten angestellt. Damit ist die Messtrecke nicht durch den vollen Durchmesser bzw. eine gesamte Erstreckung einer oben im Infusionsschlauch stehenden (nicht zu großen) Luftblase gerichtet, sondern maximal durch eine untere Sekante dieser Luftblase. Es kann auch sein, dass die Messstrecke die Luftblase nur tangiert oder sogar luftblasenaußenseitig passiert. Damit ist der Teil der Messstrecke, der durch die (nicht zu große) stehende Luftblase verläuft, (gegenüber einer Erfassung gemäß dem Stand der Technik) verkürzt, bevorzugt minimiert. Dadurch ist eine vom Luftblasensensor ermittelte Dämpfung vermindert. Dadurch kann eine Alarmschwelle einer Elektronik bzw. einer Steuereinheit, die das Signal des Luftblasensensors auswertet, unterschritten werden. Dadurch lassen sich Fehlalarme durch in der Messtrecke des Luftblasensensors stehende für den Patienten unkritische Luftblasen vermeiden.

Selbstverständlich ist der offenbarungsgemäße Luftblasensensor auch zur Erkennung von Luft im Infusionsschlauch ausgelegt, ohne dass sich Infusionsflüssigkeit in der Messstrecke befindet, wenn also z.B. wegen einer Fehlbedienung überhaupt keine Infusionsflüssigkeit gefördert wird. Dann ist selbstverständlich die vom Luftblasensensor ermittelte Dämpfung oberhalb der Alarmschwelle.

Unter dem Begriff "Luftblasen" sind auch Blasen eines anderen Gases zu verstehen.

Vorzugsweise ist der Luftblasensensor auch für eine zusätzliche Erkennung von Partikeln ausgelegt, die ebenfalls nicht in die Blutbahn des betroffenen Patienten infundiert werden dürfen.

Besonders effektiv lassen sich Fehlalarme durch in der Messtrecke des Luftblasensensors stehende Luftblasen vermeiden, wenn die Messtrecke etwa waagerecht bzw. horizontal (maximal 5° dazu angestellt) ist.

Bei bevorzugten Weiterbildungen des Luftblasensensors ist eine Unterseite der Schlauchaufnahme von einem Schlauchträger gebildet, auf dem der Infusionsschlauch abgelegt werden kann. Insbesondere ist der Schlauchträger in einem Abstand unter der Messtrecke angeordnet, der dem Radius eines Außenmantels des Infusionsschlauches entspricht.

Gemäß einer Ausgestaltung ist der Ultraschallsender relativ zum gehäusefesten Ultraschallempfänger bewegbar, oder der Ultraschallempfänger ist relativ zum gehäusefesten Ultraschallsender bewegbar.

Dann kann eine Oberseite der Schlauchaufnahme von einer Schlauchanlage gebildet sein, womit die Schlauchaufnahme dreiseitig geschlossen und seitlich geöffnet ist. Beim Verbau in einer peristaltischen Infusionspumpe bedeutet dies, dass die Schlauchaufnahme nach vorne geöffnet ist.

Gemäß einer anderen Ausgestaltung sind der Ultraschallsender und der Ultraschallempfänger beide gehäusefest und relativ zueinander fixiert.

Dazu können der Ultraschallsender und der Ultraschallempfänger mittels dem unter dem Infusionsschlauch angeordneten Schlauchträger miteinander verbunden sein, womit die Schlauchaufnahme dreiseitig geschlossen und nach oben geöffnet ist.

Bei einem Ausführungsbeispiel mit einer schrägen Messstrecke, z.B. bei einer 25 bis 45° zur Horizontalen bzw. Waagerechten angestellten Messstrecke sind der Ultraschallsender und der Ultraschallempfänger über ein ebenso schräges Verbindungteil miteinander verbunden. Auch damit sind der Ultraschallsender und der Ultraschallempfänger zur betroffenen Infusionspumpe gehäusefest, und die Schlauchaufnahme ist dreiseitig geschlossen. Insbesondere ist das Verbindungteil in einem Abstand zur Messtrecke angeordnet, der dem Radius des Außenmantels des Infusionsschlauches entspricht.

Die medizinische Infusionspumpe gemäß der Offenbarung hat ein Gehäuse und einen offenbarungsgemäßen (oder vorbeschriebenen) Luftblasensensor. Das Gehäuse oder daran befestigte Füße definiert/definieren eine Aufstandsebene, zu der die Förderrichtung etwa parallel ist, also z.B. maximal 5° angestellt. Ebenfalls mit Bezug zur Aufstandsebene ist die Messtrecke nicht senkrecht bzw. nicht vertikal, bevorzugt maximal 45°, besonders bevorzugt maximal 25° zur Aufstandsebene angestellt. Damit ist bei der üblichen waagerechten bzw. horizontalen Aufstellung der Infusionspumpe (z.B. in einem Rack) die Messtrecke nicht durch den vollen Durchmesser einer oben im Infusionsschlauch stehenden Luftblase gerichtet, sondern maximal durch eine untere Sekante der stehenden Luftblase. Es kann auch sein, dass die Messstrecke die stehende Luftblase tangiert oder passiert. Damit ist der Teil der Messstrecke, der durch die stehende Luftblase gedämpft wird, (gegenüber einer Erfassung gemäß dem Stand der Technik) verkürzt, bevorzugt minimiert. Dadurch kann eine Alarmschwelle einer Elektronik bzw. einer Steuereinheit der Infusionspumpe, die das Signal des Luftblasensensors auswertet, unterschritten werden. Dadurch lassen sich unerwünschte Alarme durch in der Messtrecke des Luftblasensensors stehende Luftblasen vermeiden.

Besonders effektiv lassen sich Fehlalarme durch stehende Luftblasen vermeiden, wenn die Messtrecke etwa parallel zur Aufstandsebene ist, also z.B. maximal 5° zur Aufstandsebene der Infusionspumpe angestellt ist.

Die Infusionspumpe kann eine Peristaltikpumpe sein, also eine Peristaltik aufweisen. In eine derartige Infusionspumpe wird ein Schlauchabschnitt des Infusionsschlauches geradlinig eingelegt, an dem mehrere äußere radial bewegliche Verdrängungskörper der Peristaltik anliegen. Mit den nacheinander radial bewegten Verdrängungskörpern wird die Infusionsflüssigkeit weitergefördert.

Die Peristaltik ist vorzugsweise von einer Frontklappe abdeckbar, wobei durch die geschlossene Frontklappe der Schlauchabschnitt des Infusionsschlauches in der Infusionspumpe gehäusefest fixierbar oder haltbar ist.

Bei einem Ausführungsbeispiel der peristaltischen Infusionspumpe ist der Ultraschallsender an der Frontklappe gebildet oder befestigt, während der Ultraschallempfänger gehäusefest (z.B. an einer Pumpenfront des Gehäuses) gebildet oder befestigt ist. Oder in kinematischer Umkehr ist der Ultraschallempfänger an der Frontklappe gebildet oder befestigt, während der Ultraschallsender gehäusefest (z.B. an der Pumpenfront) gebildet oder befestigt ist.

Bei einem anderen Ausführungsbeispiel der peristaltischen Infusionspumpe sind der Ultraschallsender und der Ultraschallempfänger beide gehäusefest. Im geschlossenen Zustand der Frontklappe ist der Ultraschallempfänger oder der Ultraschallsender benachbart zur Frontklappe angeordnet. Dann ist die Schlauchaufnahme nach oben oder schräg nach oben geöffnet.

Die Infusionspumpe kann eine Schlauchrollenpumpe sein. In eine derartige Infusionspumpe wird ein Schlauchabschnitt des Infusionsschlauches entlang einer gekrümmten Bahn eingelegt. Im Bereich der gekrümmten Bahn werden entlang dem Schlauchabschnitt eine oder mehrere äußere Rollen bewegt, wodurch die Infusionsflüssigkeit gefördert wird. Bei diesem Pumpentyp ist vorzugsweise der vorbeschriebene Luftblasensensor mit der nach oben geöffneten Schlauchaufnahme vorgesehen.

Bei beiden Pumpentypen ist der Luftblasensensor vorzugsweise stromab bzw. patientenseitig von der Peristaltik bzw. von den Rollen angeordnet.

### Kurzbeschreibung der Figuren

Figur 1 zeigt ein erstes Ausführungsbeispiel einer Infusionspumpe gemäß der vorliegenden Offenbarung;
Figur 2 zeigt einen Teil der Infusionspumpe aus Figur 1 mit einem Luftblasensensor gemäß dem Stand der Technik in einer schematischen Darstellung;
Figur 3 zeigt einen Teil der Infusionspumpe aus Figur 1 mit einem ersten Ausführungsbeispiel eines Luftblasensensors gemäß der vorliegenden Offenbarung in einer schematischen Darstellung;
Figur 4 zeigt einen Teil der Infusionspumpe aus Figur 1 mit einer ersten Variante eines zweiten Ausführungsbeispiels eines Luftblasensensors gemäß der vorliegenden Offenbarung in einer schematischen Darstellung;
Figur 5 zeigt einen wesentlichen Teil eines zweiten Ausführungsbeispiels einer Infusionspumpe gemäß der vorliegenden Offenbarung in einer schematischen Darstellung;
Figur 6 zeigt die Signalkurven des Luftblasensensors des Standes der Technik gemäß Figur 2 und der Luftblasensensoren gemäß den Figuren 3 und 4 und eines Luftblasensensors gemäß einem weiteren Ausführungsbeispiel; und
Figur 7 zeigt eine zweite Variante des zweiten Ausführungsbeispiels des Luftblasensensors gemäß der vorliegenden Offenbarung in einer schematischen Darstellung.

### Beschreibung der Ausführungsbeispiele

Nachstehend wird Stand der Technik und es werden Ausführungsbeispiele der vorliegenden Offenbarung auf der Basis der zugehörigen Figuren beschrieben.

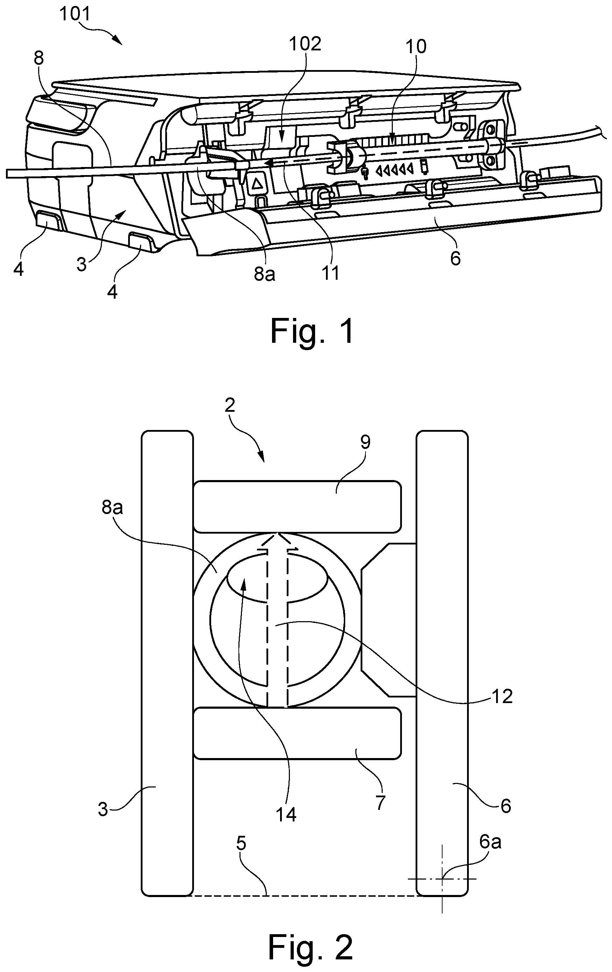

Figur 1 zeigt ein erstes Ausführungsbeispiel einer Infusionspumpe 101 gemäß der vorliegenden Offenbarung. In per se aus dem Stand der Technik bekannter Weise hat sie ein Gehäuse 3 mit vier Füßen 4, die eine Aufstandsebene der Infusionspumpe 101 definieren. In einer normalen Betriebsposition der Infusionspumpe 101 ist diese Aufstandsebene waagerecht, also senkrecht zum Schwerefeld der Erde.

An der in Figur 1 gezeigten Vorderseite des Gehäuses 3 ist eine (in Figur 1 geöffnete) Frontklappe 6 angeordnet. Hinter dieser Frontklappe 6 erstreckt sich über die volle Breite der Gehäuses 3 bzw. der Frontklappe 6 ein Schlauchabschnitt 8a eines Infusionsschlauches 8, wobei dieser Schlauchabschnitt 8a ein sogenanntes Pumpensegment aus weicherem Silikon aufweist. An diesem Pumpensegment greifen Verdrängungskörper einer Peristaltik 10 an, um die Infusionsflüssigkeit im Infusionsschlauch 8 (in Figur 1 nach links) entlang einer Förderrichtung 11 in Richtung zu einem ersten Luftblasensensor 102 und dann weiter zu einem Patientenzugang zu fördern.

Der in der Infusionspumpe 101 aufgenommene Schlauchabschnitt 8a ist parallel zu der von den Füßen 4 gebildeten Aufstandsebene. Daher sind der Schlauchabschnitt 8a und die Förderrichtung 11 in der normalen Betriebsposition der Infusionspumpe 101 ebenfalls waagerecht. Damit ergibt sich das Problem, dass sich in dem Schlauchabschnitt 8a stehende Luftblasen festsetzen können.

Figur 2 zeigt einen Luftblasensensor 2 gemäß dem Stand der Technik, der an der Infusionspumpe 101 gemäß Figur 1 verbaut sein kann. Der geschnitten dargestellte Schlauchabschnitt 8a ist zwischen einer Pumpenfront des Gehäuses 4 und der um eine Schwenkachse 6a schwenkbaren Frontklappe 6 des Gehäuses 4 eingespannt und fixiert. Der Luftblasensensor 2 hat einen Ultraschallsender 7 und einen Ultraschallempfänger 9. Eine Messtrecke 12 vom unteren Ultraschallsender 7 zum oberen Ultraschallempfänger 9 ist senkrecht zur Aufstandsebene 5 und damit senkrecht bzw. vertikal zur Erdoberfläche. Weiterhin ist die Messtrecke 12 mittig durch den Schlauchabschnitt 8a gerichtet.

In dem in den Luftblasensensor 2 eingesetzten Schlauchabschnitt 8a hat sich eine stehende Luftblase 14 festgesetzt, so dass die Messstrecke 12 mit dem größtmöglichen Streckenanteil durch die Luftblase 14 verläuft. Daher erfährt das Signal eine maximale Dämpfung. Dies kann zu einem Alarm der Infusionspumpe 101 führen, der ein unerwünschter Alarm ist, da dieser Zustand (unbewegliche Luftblase im Sensorbereich) für den Patienten unkritisch ist.

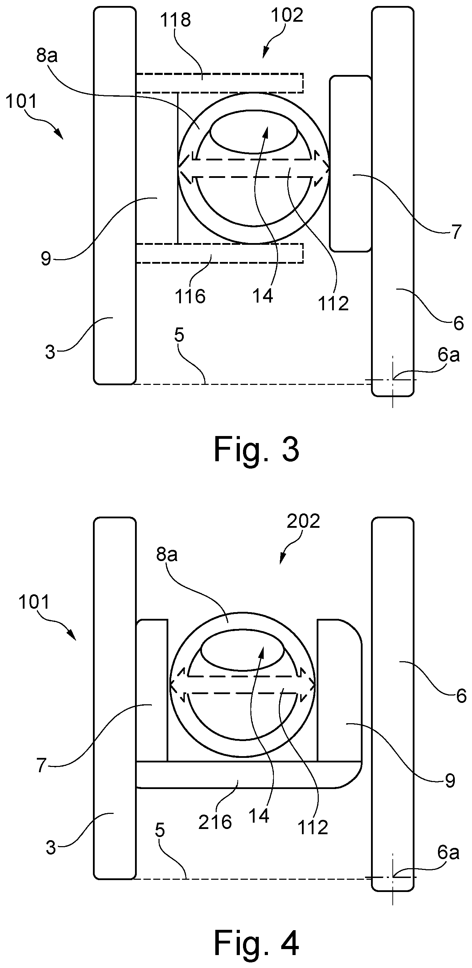

Figur 3 zeigt einen Teil des Gehäuses 3 der Infusionspumpe 101 aus Figur 1 mit dem ersten Ausführungsbeispiel des Luftblasensensors 102 gemäß der vorliegenden Offenbarung.

Hinter der (in Figur 3 geschlossenen) und um die Schwenkachse 6a schwenkbaren Frontklappe 6 des Gehäuses 4 ist der geschnitten dargestellte Schlauchabschnitt 8a in die Schlauchaufnahme des Luftblasensensors 102 eingespannt und dort fixiert. Dazu kann eine Schlauchaufnahme des Luftblasensensors 102 einen unteren Schlauchträger 116 und eine obere Schlauchanlage 118 aufweisen, wobei die Schlauchaufnahme des Luftblasensensors 102 nach vorn geöffnet ist.

Der Luftblasensensor 102 hat den Ultraschallsender 7 und den Ultraschallempfänger 9, die bei geschlossener Frontklappe 6 auf gleicher Höhe bzw. mit gleichem Abstand zur Aufstandsebene 5 der Infusionspumpe 101 angeordnet sind. Der Ultraschallsender 7 ist an einer Innenseite der Frontklappe 6 befestigt und daher um die Schwenkachse 6a bewegbar. Der Ultraschallempfänger 9 ist an der Pumpenfront des Gehäuses 3 befestigt. Damit ist die Messtrecke 112 waagerecht zur Aufstandsebene 5 und in einer normalen Betriebsposition der Infusionspumpe 101 waagerecht bzw. horizontal zur Erdoberfläche.

Weiterhin ist bei gezeigtem Ausführungsbeispiel die Messtrecke 112 mittig oder in einem weiteren (nicht gezeigten) Ausführungsbeispiel in vertikaler Richtung versetzt zur Mitte durch den Schlauchabschnitt 8a gerichtet.

Figur 4 zeigt einen Teil des Gehäuses 3 der Infusionspumpe 101 aus Figur 1 mit einer ersten Variante eines zweiten Ausführungsbeispiels des Luftblasensensors 202 gemäß der vorliegenden Offenbarung. Hinter der (in Figur 4 geschlossenen) und um die Schwenkachse 6a schwenkbaren Frontklappe 6 des Gehäuses 4 ist der geschnitten dargestellte Schlauchabschnitt 8a in die Schlauchaufnahme eingespannt und fixiert.

Die Schlauchaufnahme ist nach oben geöffnet, so dass der Schlauchabschnitt 8a nach dem Öffnen der Frontklappe 6 von oben in die Schlauchaufnahme eingelegt und vorzugsweise dort fixiert wird. Durch das Schließen kann der Schlauchabschnitt 8a alternativ oder ergänzend auch an anderer Stelle gehäusefest fixiert werden.

Der Luftblasensensor 202 hat den Ultraschallsender 7 und den Ultraschallempfänger 9, die beide auf gleicher Höhe bzw. mit gleichen Abstand zur Aufstandsebene 5 der Infusionspumpe 101 angeordnet sind, so dass die Messtrecke 112 waagerecht/ parallel zur Aufstandsebene 5 und damit waagerecht bzw. horizontal bzw. parallel zur Erdoberfläche gerichtet ist.

Der Ultraschallsender 7 und der Ultraschallempfänger 9 sind beide gehäusefest. Dazu sind sie über eine Verbindungsteil 216 aneinander fixiert, das unter dem Schlauchabschnitt 8a angeordnet ist und daher als Schlauchträger 216 wirken kann.

Weiterhin ist beim in Figur 4 gezeigten Ausführungsbeispiel die Messtrecke 112 mittig durch den Schlauchabschnitt 8a gerichtet.

Mit Blick auf die Figuren 3 und 4 hat sich in dem in den Luftblasensensor 102; 202 eingesetzten Schlauchabschnitt 8a eine stehende Luftblase 14 festgesetzt, so dass die Messstrecke 112 ähnlich einer Sekante (nur) durch einen unteren Bereich der Luftblase 14 verläuft. Daher erfährt das Signal eine gegenüber dem Stand der Technik verringerte Dämpfung, die offenbarungsgemäß unter einer Alarmschwelle der Infusionspumpe 101 bleibt, da dieser Zustand für den Patienten unkritisch ist.

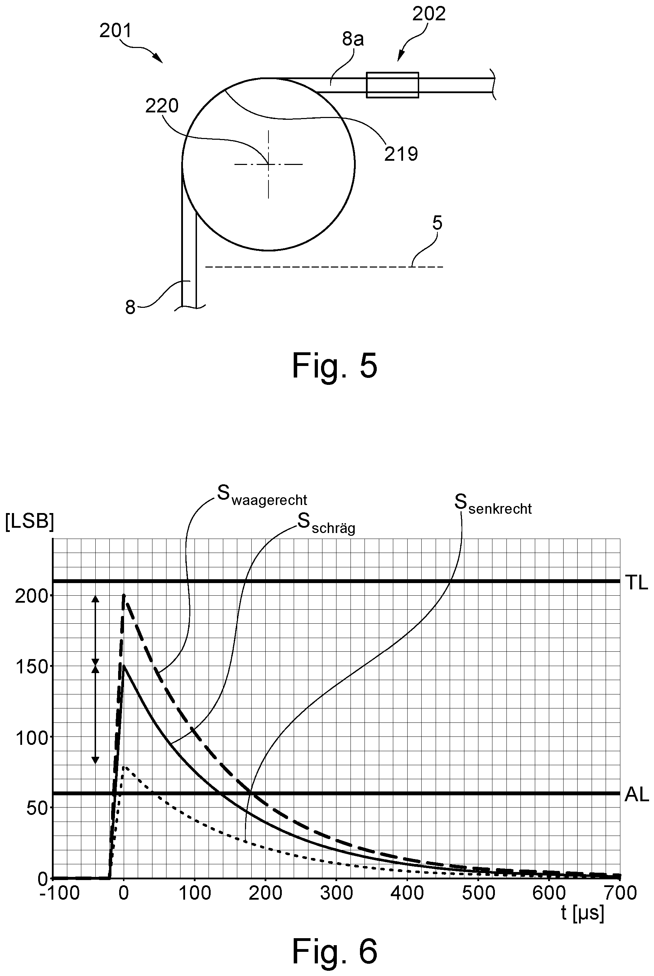

Figur 5 zeigt wesentliche Teile eines zweiten Ausführungsbeispiels einer Infusionspumpe 201 gemäß der vorliegenden Offenbarung mit dem Luftblasensensor 202 aus Figur 4. Die Infusionspumpe 201 ist als Schlauchrollenpumpe ausgeführt, in die ebenfalls ein Schlauchabschnitt 8a eines Infusionsschlauches 8 eingelegt wird. Ein Teil des in der Schlauchrollenpumpe 201 eingelegten Schlauchabschnitts 8a wird entlang einer Kurvenbahn 219 gekrümmt. Entlang der Innenseite des auf diese Weise gekrümmten Teils des Schlauchabschnitts 8a laufen (nicht gezeigte) Rollen und dabei um eine Achse 220 um. Damit wird die Infusionsflüssigkeit durch den Teil des Schlauchabschnitts 8a gefördert.

Ein stromab des gekrümmten Teils angeordneter Teil des in der Schlauchrollenpumpe 201 eingelegten Schlauchabschnitts 8a erstreckt sich parallel zur Aufstandsebene 5 des (nicht näher gezeigten) Gehäuses und ist daher in einer normalen Betreibposition der Schlauchrollenpumpe 201 waagerecht angeordnet. An diesem Teil ist das in Figur 4 gezeigte Ausführungsbeispiel des Luftblasensensors 202 angeordnet, das den gehäusefesten Ultraschallsender 7 und den gehäusefesten Ultraschallempfänger 9 aufweist und dessen Schlauchaufnahme nach oben offen ist.

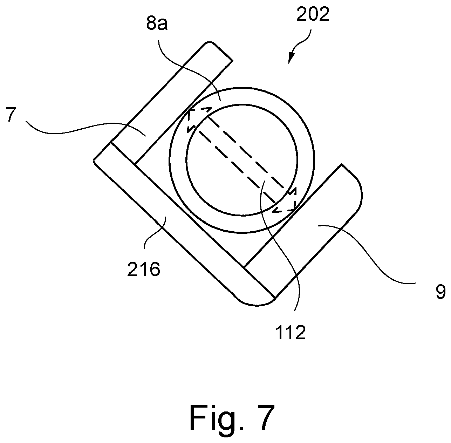

Figur 6 zeigt drei Signalverläufe über die Zeit, die entstehen, wenn die in den Figuren 2 bis 4 gezeigte stehende Luftblase 14 von den verschiedenen Luftblasensensoren 2; 102; 202 gemäß den Figuren 1 bis 5 und gemäß Figur 7 erfasst wird. Genauer gesagt ist ein Komparator-Eingangssignal S_{senkrecht} des Luftblasensensors 2 des Standes der Technik aus Figur 2 gezeigt. Weiterhin ist ein Komparator-Eingangssignal S_{waagerecht} des Luftblasensensors 102 aus Figur 3 und des Luftblasensensors 202 aus Figur 4 gezeigt. Schließlich ist ein Komparator-Eingangssignal S_{schräg} eines Luftblasensensors gemäß Figur 7 gezeigt, bei dem die Messtrecke 12 um 45° geneigt ist. Bei einer 45° Drehung des Luftblasensensor 102; 202 wird die dargestellte Luftblase zu ca. 90% "getroffen". Dazu kann auch der Luftblasensensor 102 aus Figur 3 gegenüber der Anordnung aus Figur 3 um 45° im Uhrzeigersinn um eine Längsachse des Schlauchabschnitts 8a um 45° gekippt bzw. gedreht sein. Oder der Luftblasensensor 202 aus Figur 4 wird gemäß Figur 7 gegenüber der Anordnung aus Figur 4 um eine Längsachse des Schlauchabschnitts 8a um 45° im Uhrzeigersinn gekippt bzw. gedreht.

Auch mit dem Komparator-Eingangssignal S_{schräg} ist die offenbarungsgemäß abgeschwächte Dämpfung des Luftblasensensors 202 gemäß der zweiten Variante des zweiten Ausführungsbeispiels aus Figur 7 gegenüber dem Luftblasensensor 2 des Standes der Technik gezeigt.

Insbesondere mit dem Komparator-Eingangssignal S_{waagerecht} ist die offenbarungsgemäß noch weiter (stärker) abgeschwächte Dämpfung der Luftblasensensoren 102, 202 gemäß den Ausführungsbeispielen aus den Figuren 3 und 4 gegenüber dem Luftblasensensor 2 des Standes der Technik gezeigt.

Figur 7 zeigt eine zweite Variante des zweiten Ausführungsbeispiels des Luftblasensensors 202 gemäß der vorliegenden Offenbarung. Hinter der (nur in Figur 4 gezeigten) und um die Schwenkachse 6a schwenkbaren Frontklappe 6 des Gehäuses 4 ist der geschnitten dargestellte Schlauchabschnitt 8a in die Schlauchaufnahme aufgenommen. Die Schlauchaufnahme ist nach schräg oben und vorne geöffnet. Die (nur in Figur 4 gezeigte) Luftblase ist (aus Gründen ihrer geringen Dichte) vertikal oben.

### Bezugszeichenliste:

- 2: Luftblasensensor des Standes der Technik
- 3: Gehäuse
- 4: Fuß
- 5: Aufstandsebene
- 6: Frontklappe
- 6a: Schwenkachse
- 7: Ultraschallsender
- 8: Infusionsschlauch
- 8a: Schlauchabschnitt mit waagerechter Förderrichtung
- 9: Ultraschallempfänger
- 10: Peristaltik
- 11: Förderrichtung
- 12; 112: Messstrecke
- 101: peristaltische Infusionspumpe
- 102: Luftblasensensor (nach vorne offen)
- 14: stehende Luftblase
- 116: Schlauchträger
- 118: Schlauchanlage
- 201: Infusionspumpe / Schlauchrollenpumpe
- 202: Luftblasensensor (nach oben offen)
- 216: Schlauchträger / Verbindungsteil
- 220: Achse

- S_{senkrecht}: Signal bei senkrechter Messstrecke gemäß dem Stand der Technik
- S_{waagerecht}: Signal bei waagerechter Messstrecke
- S_{schräg}: Signal bei schräger Messstrecke

## Patentansprüche

1. Luftblasensensor (102; 202) mit einem Ultraschallsender (7) und einem Ultraschallempfänger (9) und mit einer Schlauchaufnahme, die sich entlang einer etwa waagerechten und/oder horizontalen Förderrichtung (11) erstreckt, wobei der Ultraschallsender (7) an einer ersten Seite der Schlauchaufnahme angeordnet ist und/oder eine erste Seite der Schlauchaufnahme bildet, und wobei der Ultraschallempfänger (9) an einer der ersten Seite gegenüber liegenden zweiten Seite angeordnet ist und/oder eine zweite Seite der Schlauchaufnahme bildet, wobei eine vom Ultraschallsender (7) zum Ultraschallempfänger (9) gerichtete Messtrecke (112) nicht senkrecht bzw. nicht vertikal ist, vorzugsweise maximal 45°, besonders bevorzugt maximal 25° zur Horizontalen und/oder Waagerechten angestellt ist.

2. Luftblasensensor (102; 202) nach Anspruch 1, wobei die Messtrecke (112) etwa waagerecht bzw. horizontal ist.

3. Luftblasensensor (102; 202) nach einem der vorhergehenden Ansprüche, wobei eine Unterseite der Schlauchaufnahme von einem Schlauchträger (116; 216) gebildet ist.

4. Luftblasensensor (102) nach einem der vorhergehenden Ansprüche, wobei der Ultraschallsender (7) relativ zum Ultraschallempfänger (9) bewegbar ist, oder wobei der Ultraschallempfänger (9) relativ zum Ultraschallsender (7) bewegbar ist.

5. Luftblasensensor (102) nach den Ansprüchen 3 und 4, wobei eine Oberseite der Schlauchaufnahme von einer Schlauchanlage (118) gebildet ist, womit die Schlauchaufnahme dreiseitig geschlossen und seitlich geöffnet ist.

6. Luftblasensensor (202) nach einem der Ansprüche 1 bis 3, wobei der Ultraschallsender (7) und der Ultraschallempfänger (9) relativ zueinander fixiert sind.

7. Luftblasensensor (202) nach den Ansprüchen 3 und 6, wobei der Ultraschallsender und der Ultraschallempfänger über den Schlauchträger (216) miteinander verbunden sind, womit die Schlauchaufnahme dreiseitig geschlossen und nach oben geöffnet ist.

8. Luftblasensensor (202) nach Anspruch 1, wobei die Messstrecke 25° bis 45° zur Horizontalen und/oder Waagerechten angestellt ist, und wobei der Ultraschallsender (7) und der Ultraschallempfänger (9) über ein Verbindungteil (216) miteinander verbunden sind, womit die Schlauchaufnahme dreiseitig geschlossen ist.

9. Medizinische Infusionspumpe (101; 201) mit einem Gehäuse (3) und mit einem Luftblasensensor (102; 202) nach einem der vorhergehenden Ansprüche, wobei das Gehäuse (3) oder daran befestigte Füße (4) eine Aufstandsebene (5) definiert/definieren, zu der die Förderrichtung (11) etwa parallel ist, und zu der die Messtrecke (112) nicht senkrecht bzw. nicht vertikal ist, vorzugsweise maximal 45°, besonders bevorzugt maximal 25° zur Aufstandsebene (5) angestellt ist.

10. Medizinische Infusionspumpe (101; 201) nach Anspruch 9, wobei die Messtrecke (112) etwa parallel, vorzugsweise parallel, zur Aufstandsebene (5) ist.

11. Medizinische Infusionspumpe (101) nach Anspruch 9 oder 10 mit einer Peristaltik (10).

12. Medizinische Infusionspumpe (101) nach Anspruch 11, wobei die Peristaltik (10) von einer Frontklappe (6) abdeckbar ist, und wobei durch die geschlossene Frontklappe (6) ein Schlauchabschnitt (8a) des Infusionsschlauches (8) in der medizinischen Infusionspumpe (101) fixierbar oder haltbar ist.

13. Medizinische Infusionspumpe (101) nach Anspruch 12, wobei der Ultraschallsender (7) an der Frontklappe (6) gebildet oder befestigt ist, während der Ultraschallempfänger (9) gehäusefest ist, oder wobei der Ultraschallempfänger (9) an der Frontklappe (6) gebildet oder befestigt ist, während der Ultraschallsender (7) gehäusefest ist.

14. Medizinische Infusionspumpe (101) nach Anspruch 12, wobei im geschlossenen Zustand der Frontklappe (6) der Ultraschallempfänger (9) oder der Ultraschallsender (7) benachbart zur Frontklappe (6) angeordnet ist.

15. Medizinische Infusionspumpe (201) nach Anspruch 9 oder 10, die eine Schlauchrollenpumpe ist.
